# EUROPÄISCHE PATENTSCHRIFT

(11) **EP 2 086 437 B1**
(45) Veröffentlichungstag und Bekanntmachung des Hinweises auf die Patenterteilung: **18.06.2014**
(21) Anmeldenummer: 06818704.6
(22) Anmeldetag: 21.11.2006
(51) Int. Cl.: A61B 17/72

(54) **Implantat für Röhrenknochen**
Implant for tubular bones
Implant pour os tubulaires

(43) Veröffentlichungstag der Anmeldung: 12.08.2009
(73) Patentinhaber: Pieske, Oliver, 82166 Gräfelfing (DE)
(72) Erfinder: Pieske, Oliver, 82166 Gräfelfing (DE)
(74) Vertreter: Hoeger, Stellrecht & Partner Patentanwälte
(86) Internationale Anmeldenummer: PCT/EP2006/011151
(87) Internationale Veröffentlichungsnummer: WO 2008/061543

(56) Entgegenhaltungen:
- WO-A-98/26725
- WO-A-98/49962
- WO-A-2004/014243
- WO-A-2004/089255
- DE-A- 2 657 303
- US-A- 5 053 035
- US-A- 6 045 551
- US-A1- 2005 027 294
- DATABASE WPI Week 198512 Derwent Publications Ltd., London, GB; AN 1985-073545 XP002443905 -& SU 1 111 748 A (KIEV MEDICAL INST) 7. September 1984 (1984-09-07)
- DATABASE WPI Week 198004 Derwent Publications Ltd., London, GB; AN 1980-A8866C XP002443987 & SU 662 082 A (TARTUS UNIV) 15. Mai 1979 (1979-05-15)

## Beschreibung

Die vorliegende Erfindung betrifft ein Implantat gemäß dem Oberbegriff des Anspruchs 1.

Die Klavikulafraktur, d. h. der Schlüsselbeinbruch, ist eine der häufigsten Frakturen des Menschen (20 bis 25 % aller kindlichen und 5 bis 15 % aller erwachsenen Frakturen). Etwa 80% der Klavikulafrakturen sind Schaftfrakturen. Üblicherweise werden diese Frakturen konservativ mit circa 6-wöchiger Rucksackverband-Ruhigstellung behandelt. Neben den zum Teil erheblich störenden Nebenwirkungen des Rucksackverbandes, z. B Störung der Durchblutung/Neurologie durch Plexuskompression, sind beim Erwachsenen jedoch teilweise sowohl die funktionellen als auch kosmetischen Langzeitergebnisse durch Verheilung in Fehlstellung ("mal-union") oder Pseudarthrose ("non-union") und prominenter Kallusbildung unbefriedigend. Bei Klavikulaschaftfrakturen mit initial ausgeprägter Fehlstellung wird daher zunehmend die operative Stabilisierung durchgeführt. Dabei kommt als "golden standard" die Plattenosteosynthese zur Anwendung. Alternativ kann die Klavikulafraktur mit einem intramedullären Nagel versorgt werden. Die Vor- und Nachteile der jeweiligen Osteosyntheseform werden in der folgenden Tabelle dargestellt:

| Implantat | Vorteile | Nachteile |
|---|---|---|
| Plattenosteosynthese | - hohe Primärstabilität | - Große Wunde (mindestens 3 Schrauben je Frakturseite → Wundlänge > 6 cm) |
| | - anatomische Reposition zumeist möglich | - große Devastierung im Frakturbereich und damit Störung der Frakturheilung und Pseudarthrosegefahr |
| | | - daher Metallentfernung frühestens nach 18 Monaten möglich |
| | | - extramedulläre Kraftübertragung |
| | | - Platte direkt unter der Haut wird zumeist als störend und bei bestimmten Tätigkeiten als schmerzhaft empfunden: z.B. Rucksacktragen, Akkordeonspielen etc. |
| | | - Metallentfernung nur in Vollnarkose möglich, mit wiederum entsprechend großem operativen Trauma und erniedrigtem Patientenkomfort |
| Intramedullärer Nagel (z.B. TEN oder Thalon) | - kleines operatives Trauma: kleine Inzision mediale Klavikula, gegebenenfalls zusätzlicher Schnitt über Frakturbereich zur offenen Reposition | - geringere Primärstabilität, insbesondere keine Kompression auf die Fraktur |
| | | - Gefahr der sekundären Dislokation und Pseudarthrose ("non-union") und Implantatwanderung (teilweise lebensgefährlich!) |
| | | - Metallentfernung zumeist nur in Vollnarkose möglich, mit wiederum entsprechend großem operativen Trauma und erniedrigtem Patientenkomfort |

Um über einen minimal invasiven Zugang unter Erhalt der Vaskularisation im Frakturbereich eine hohe Primärstabilität zu erzielen und darüber hinaus auch eine Metallentfernung in Lokalanästhesie zu ermöglichen, soll ein völlig neuartiges Osteosyntheseprinzip entwickelt werden, das insbesondere bei der Klavikulafraktur aber prinzipiell auch an anderen Röhrenknochen zur Anwendung kommen kann.

Es gibt bereits fortschrittliche Ansätze in der Verwendung von nicht starren Verbindungselementen für die Behandlung von Gelenkverletzungen.

Ein vereinfachtes gelenküberbrückendes Implantat bei gelenknahen Knochenbrüchen, aber insbesondere bei Gelenkverrenkungen ist in der DE 36 30 138 A1 dargestellt. In der DE 36 30 138 A1 werden dabei Stahlseile verwendet, um eine elastische Verbindung von Gelenkteilen zu ermöglichen. Es sind zwei Ankerteile vorgesehen, von denen jeweils eines am Knochen- bzw. Gelenkteil befestigt und beide Ankerteile miteinander gelenkig verbunden sind, wobei die Verbindung von mindestens einem Seil gebildet ist. Die DE 36 30 138 A1 zielt jedoch lediglich auf eine flexible Verbindung bei Gelenkfrakturen ab, wobei eine starre Verbindung von einem gebrochenen Röhrenknochen nicht offenbart wird. Das Implantat ist nicht resorbierbar und nicht minimal-invasiv zu implantieren.

In DE 196 18 552 A1 wird für ein gelenküberbrückendes Implantat vorgeschlagen, ein Knochensegment durch eine Zugkraft anzutreiben, die durch Beugebewegung des im Gelenk angelenkten gesunden Knochens in einem Seil erzeugt und von diesem intermedulär durch einen Marknagel unmittelbar oder mittelbar am Segment zum Angriff gebracht wird, um das Segment zur Überbrückung eines Knochendefekts an einem Röhrenknochen autoinduktiv mit einer vollständigen implantierten Vorrichtung zu transportieren. Zur Stabilisierung des intramedullären Systems werden Querbolzen benötigt. Die Problemstellung und der vorgeschlagene Lösungsansatz sind jedoch ebenfalls nicht ohne weiteres auf den Fall von glatt gebrochenen Knochen übertragbar. Zudem ist das Implantat nicht resorbierbar und nicht minimal-invasiv zu implantieren.

Die DE 1 852 874 U zeigt ein Implantat zum Verbinden zweier Teile eines gebrochenen Röhrenknochens, wobei das Implantat durch ein Formstück gebildet ist, das dazu bestimmt ist, in den Markraum eines gebrochenen Röhrenknochens eingeführt zu werden. Das Formstück weist eine Durchgangsöffnung mit einem konstanten Innendurchmesser aus, die sich durch das gesamte Formstück erstreckt. Das Implantat muss ohne Spannung auskommen, womit keine Frakturkompression erreicht wird. Zudem werden Querbolzen zur Stabilisierung des intramedullären Systems benötigt Außerdem offenbart die DE 1 852 874 U kein resorbierbares Material und keine minimal-invasive Implantation.

US 6 045 551 offenbart ein quer zur Knochenachse verlaufendes Implantat für die zugfeste Verbindung zweier Teile eines gebrochenen Röhrenknochens, wobei das Implantat zwei Ankerteile, ein Seil und ein wahlweise resorbierbares Formstück aufweist, wobei die Ankerteile an dem einen Seil befestigt werden können, wobei das Formstück eine Durchgangsöffnung aufweist, durch die das Seil geführt wird, so dass sich das Formstück zwischen zwei Ankerteilen um das Seil erstreckt. Da mehrere Seilanker benötigt werden, wird das Implantat nicht minimal-invasiv implantiert und es kommt zu keiner Vereinfachung der Operation und zu einer Verkürzung der Operationszeit. Der Oberbegriff des Anspruchs 1 basiert auf dieser Offenbarung.

DE 824 377 betrifft eine nichtresorbierbare Vorrichtung zum Zusammenziehen der Bruchenden eines gebrochenen Röhrenknochens mit Hilfe eines in einem Abstand von der Bruchstelle einzuschiebenden Zugliedes mit einem bei einem Zuge sich an der Knochenwand festsetzenden Anker. Jedoch offenbart DE 824 377 kein röhrenförmiges Implantat, dass eine translatorische Dislokationstendenz der Knochenenden verhindern könnte.

Die EP 0 428 985 A1 beschreibt einen Marknagel der durch eine Drahtschleife in einem Röhrenknochen fixiert werden kann, wobei das primäre Ziel der EP 0 428 985 A1 die vereinfachte distale Verriegelung ist. Sowohl im körpernahen als auch körperfemen Knochenbruchende müssen jedoch zur Stabilisierung des intramedullären Systems grundsätzlich Querbolzen eingebracht werden, welche, ebenso wie der Marknagel selbst, nicht resorbierbar sind.

Weitere bioresorbierbare Systeme zur Einbringung in den Knochen werden in der US 6,096,060 beschrieben, die ein Ankersystem zur Befestigung von weichem Gewebe am Knochen beschreibt und in der US 5,470,334, welche eine Knochenfixierungsschraube beschreibt. Beide Systeme sind jedoch nicht zur Behandlung von Klavikulafrakturen geeignet.

Die WO 98/49962 A1 offenbart ein resorbierbares intramedulläres Implantat und Verfahren zu dessen Verwendung. In der WO 2004/014243 A1 sind ein Verfahren sowie eine Vorrichtung zur Behandlung des Mittelfußbereichs mit einem intermedulären Nagel beschrieben. Die WO 2004/089255 A1 offenbart eine Vorrichtung zur temporären Schienung von Zehen.

Es ist daher wünschenswert, ein Implantat zur Verfügung zu haben, welches es gestattet, die Bruchstücke eines Röhrenknochens mit geringerem Aufwand als bisher durch ein Implantat zu stabilisieren.

Es ist weiterhin wünschenswert, ein Implantat zur Verfügung zu haben, dass es gestattet die Entfernung des Implantats nach biologischer Durchbauung wesentlich zu vereinfachen.

Es ist erstrebenswert, eine Operationstechnik zur Verfügung zu haben, die es gestattet, einen Eingriff zum Implantieren und zum Entfernen eines Implantats zu vereinfachen.

Es ist weiterhin, wie in allen operativen Verfahren erstrebenswert, ein schonendes Operieren mit nur geringem Blutverlust und kleiner Wundfläche zu erreichen, und trotzdem eine stabile Bruchversorgung zu erreichen.

Diese Aufgabe wird bei einem Implantat der eingangs beschriebenen Art erfindungsgemäß gelöst durch die Merkmale des kennzeichnenden Teils des Anspruchs 1. Weitere vorteilhafte Ausgestaltungen werden durch die abhängigen Ansprüche definiert.

Gemäß vorliegenden Erfindung wird ein Implantat für die zugfeste Verbindung zweier Teile eines mehr oder weniger glatt gebrochenen Röhrenknochens bereitgestellt. Das Implantat umfasst dabei mindestens zwei Ankerteile, mindestens ein Seil und ein Formstück. Die Ankerteile können dabei an dem mindestens einen Seil befestigt werden. Es ist jedoch auch vorgesehen, dass ein Ankerteil bereits von vornherein an dem Seil befestigt ist. Das Formstück ist mit mindestens einer Durchgangsöffnung versehen, durch die das Seil geführt wird, sodass sich das Formstück zwischen den zwei Ankerteilen um das Seil erstreckt.

Mit der vorliegenden Erfindung soll das Prinzip des innen verspannten Stapels genutzt werden, um die Bruchflächen eines Röhrenknochens gegeneinander zu pressen. Dabei wird ausgenutzt, dass bei einem Markknochen im Falle eines glatten Bruchs bei einer Knickung sich ein in dem Markraum verlaufendes Seil verlängern würde. Ist das Seil gespannt, kann es sich nicht weiter verlängern, und die Knickbewegung kann verhindert werden.

Die Ankerstücke sind dazu bestimmt, das Seil an dem Knochen zu fixieren. Die Ankerstücke können in einer einfachen Ausführungsform einfach nur als Verdickungen oder Endnippel ausgeführt sein, wie es von "Fahrrad-Bowdenzügen" bekannt ist. Wenn das Seil durch ein kleines Loch oder einen schmalen Schlitz in den Markraum eines Knochens geführt wird, kann eine Verdickung oder ein Nippel am Ende das Seil sicher am Knochen verankern. Es ist jedoch ebenfalls möglich, beispielsweise geschraubte Ankerplatten, einen Schraubanker oder eine kleine (gegebenenfalss resorbierbare) Platte (vergleichbar einem Hemdknopf) mit mindestes einem Loch, durch welche das Seil gefädelt und befestigt wird zu verwenden. Die Verwendung komplexerer Verankerungsmittel als beispielsweise Nippel ist jedoch nicht unbedingt notwendig. Bei komplexeren Brüchen ist es ebenfalls denkbar, eine verschraubte Platte an einem Trümmerbruchabschnitt als Ankerstück zu verwenden, und einen (im Wesentlichen) glatten Bruch in einem anderen Abschnitt mit dem Implantat der vorliegenden Erfindung zu stabilisieren. Ein Ankerstück kann an dem Seil lösbar befestigt sein.

Es ist ebenfalls zu bemerken, dass das Seil mit jeweils einem Ende von einem Ankerteil gehalten wird und einfach oder mehrfach verlegt sein kann. Es ist damit prinzipiell auch möglich mehrere Implantate an einem Bruch zu verwenden, was die Stabilisierung selbst größerer Hohlknochen ermöglichen kann.

In einer beispielhaften Ausführungsform ist das Seil ein Stahlseil, das aus Einzelfasern geflochten bzw. geschlagen ist. Es sollte nicht notwendig sein, zu erwähnen, dass die verwendeten Materialien, die für die Komponenten des Implantats (bzw. dessen Oberflächen) verwendet werden, physiologisch verträglich sein sollen bzw. müssen. Es ist ebenfalls zu überlegen, andere Materialien und/oder Metalle zu verwenden. Es kann in Betracht gezogen werden, für das Seil einen Kunststoff oder ein resorbierbares Material zu verwenden. Es kann ebenfalls in Betracht gezogen werden, für das Seil beispielsweise Titan oder Platin zu verwenden.

In einer anderen beispielhaften Ausführungsform der vorliegenden Erfindung ist das Formstück im Wesentlichen zylinderförmig. Durch ein zylinderförmiges Formstück kann beispielsweise ein extrudierter Schlauch als Formstück verwendet werden. In der zylinderförmigen Ausführungsform kann das Formstück dazu dienen, das Seil weiter von dem Dreh- bzw. Knickpunkt des Röhrenknochens entfernt zu halten, was es gestattet, das Seil dünner auszuführen.

Es ist ebenfalls in Betracht zu ziehen, das Formstück als einen Zylinder mit nicht kreisförmigem Querschnitt auszuführen. Damit könnte beispielsweise dieses Implantat für nicht kreisförmige Röhrenknochen verwendet werden. Das zylinderförmige Formstück ist mit abgerundeten oder konisch verlaufenden Enden versehen.

In einer weiteren exemplarischen Ausführungsform ist das Formstück im Wesentlichen spindelförmig ausgeführt. Damit kann es erleichtert werden, das Formstück zwischen an den Bruchflächen in den Markraum des mit dem Implantat zu versehenden Knochens einzuführen.

Es kann ebenfalls in Betracht gezogen werden, den größten Durchmesser des im Wesentlichen spindelförmigen Formstücks an der dicksten Stelle an den Innendurchmesser des Knochens an der Bruchstelle anzupassen. Es ist ebenfalls möglich, die Form der Querschnittsfläche an dem größten Durchmesser des im Wesentlichen spindelförmigen Formstücks an die Form der Querschnittsfläche des Hohlraums des Knochens an der Bruchstelle anzupassen. Dies könnte beispielsweise durch die Verwendung von verschiedenen vorgeformten oder speziell zugeschnittenen Formkörpern erreicht werden. Die Auswahl oder die Fertigungsdaten können anhand von bildgebenden Verfahren aus einer tomografischen Durchleuchtungsvorrichtung gewonnen werden. Durch die Spindelform kann der Formkörper an der Bruchstelle die Bruchflächen quer zur Längsrichtung des Knochens ausrichten.

Der Formkörper kann an der äußeren Oberfläche mit einer Struktur, insbesondere mit Längsrillen oder Querrillen versehen sein. Durch Längsrillen erhält der Formkörper einen im Wesentlichen sternförmigen Querschnitt. Durch die Rillen oder Stege kann sich der Formkörper an der Innenseite der Knochen verzahnen. Durch das gegenseitige Eingreifen des Formstücks in die Innenseite können Torsionskräfte an der Bruchstelle aufgenommen bzw. neutralisiert werden.

Das Formstück ist aus einem resorbierbaren Material geformt. Damit wird es ermöglicht, dass der Formkörper selbst dann im Röhrenknochen verbleibt, wenn das Seil und die Verankerungsstücke bereits wieder entfernt wurden. Durch die Auswahl eines resorbierbaren Materials wird keine Langzeitverträglichkeit des Formkörpermaterials erforderlich. Der Formkörper muss darüber hinaus nicht aus dem Körper entfernt werden, da der Körper selber diese Aufgabe übernimmt. Die Auswahl der Resorptionsgeschwindigkeit des Formkörpers sollte so gewählt werden, dass der Formkörper sich nicht oder nicht wesentlich schneller auflöst, als die Knochenmasse zusammenwächst. Die Auswahl des Materials des Formkörpers kann auch direkt auf das Alter bzw. die Geschwindigkeit des Stoffwechsels des jeweiligen Patienten abgestimmt werden.

In einer besonderen Ausführungsform ist das resorbierbare Material ausgewählt aus der Gruppe umfassend Polyglycolid (PGA), Glycolidcopolymere, Glycolide-/lactidcopolymere (PGA/PLA), Glycolid/trimethylencarbonatcopolymere (PGA/TMC) (Produkt GORE SEAMGUARD® der bywass GmbH), Stereoisomere und Copolymere von Polylactid, Poly-L-lactid (PLLA) (Produkt Sculptra® der Firma Sanofi Aventis), Poly-D-lactid (PDLA), Poly-DL-lactid (PDLLA), L-Lactid/DL-lactidcopolymere, L-Lactid/D-lactidcopolymere, Lactid/tetramethylenglycolidcopolymere, Lactid/trimethylencarbonatcopolymere, Lactid/δ-valerolactoncopolymere, Lactid/ε-caprolactoncopolymers, Polydepsipeptid (Glycin-DLlactidcopolymer), Polylactid/ethylenoxidcopolymere, asymmetrisch 3,6-substituierte Poly-1,4-dioxan-2,4-dione, Poly-β-hydroxybutyrat (PHBA), PHBA/β-hydroxyvaleratcopolymere (PHBA/PHVA), Poly-β-hydoxypropionat (PHPA), Poly-β-dioxanon (PDS), Poly-Δ-valerolacton, Poly-Δ-caprolacton, Methylmethacrylat-N-vinylpyrrolidoncopolymere, Polyesteramide, Polyester von Oxalsäure, Polydihydropyrane, Polyalkyl-2-cyanoacrylate, Polyurethane (PU), Polyvinylalkohol (PVA), Polypeptide aus α-Aminosäuren, Poly-β-maleinsäure (PMLA), Poly-β-alkansäuren, Polyethylenoxid (PEO), Chitinpolymere, Calciumhydroxidapatit oder Tricalciumphosphat (Prokukt Biobase® der Firma Zimmer dental).

Der Formkörper kann aus einem biologisch abbaubaren und resorbierbaren chirurgischen Material bestehen, dass molekular orientiert sein und eine Biegefestigkeit von 1,6 x 10³ bis 2,5 x 10⁵ kg/cm², einen Biegemodul von 5,5 x 10² bis 24,0 x 10² kg/mm² aufweisen kann. Es ist möglich, dass das Material des Formteils eine Kristallinität von 10 bis 60 % und ein Viskositätsmittel des Molekulargewichts aufweist, das vor einem Schmelzverarbeiten 300.000 bis 600.000 beträgt und nach einem Schmelzverarbeiten 200.000 bis 400.000 beträgt. Das Gewichtsverhältnis von Milchsäure/Glykolsäure des Copolymers kann dabei von 99:1 bis 75:25 betragen.

In noch einer weiteren beispielhaften Ausführungsform der vorliegenden Erfindung ist das Formstück aus einzelnen schlauch- bzw. spindelförmigen Abschnitten zusammengesetzt, die zumindest abschnittsweise ineinander geschoben sind. Dadurch kann ein einzelner beispielsweise spindelförmiger Formkörper aus einzelnen schlauchförmigen Abschnitten zusammengesetzt werden. Es ist ebenfalls möglich, aus mehreren verschiedenen übereinander geschobenen resorbierbaren Schläuchen (d.h. Formkörpern) einen "Bausatz" für unterschiedliche Röhrenknochen bereit zu stellen.

In einer anderen beispielhaften Ausführungsform ist der Formkörper aus Abschnitten aus resorbierbarem Material hergestellt, die unterschiedliche Resorptionsgeschwindigkeiten aufweisen. Damit kann angestrebt werden, dass sich der Formkörper beispielsweise durch eine langsam resorbierbare Außenschicht während dem Knochenwachstum langsam auflöst. Wenn die Außenschicht aufgelöst ist, kann sich der innere Kern nach dem Verheilen des Knochens schnell auflösen.

Das Seil kann ebenfalls in das Material des Formstücks eingegossen sein. Dies kann die Verwendung des Implantats stark vereinfachen, da der Operierende oder der Verwender des Implantats nicht mehr vergessen kann, den Formkörper auf das Seil zu fädeln.

In einer weiteren beispielhaften Ausführungsform des Implantats weist das Formteil mindestens zwei weitere Löcher auf, die sich im Wesentlichen quer zu der Längsrichtung des Formstücks erstrecken. Dadurch kann ein in der Kortikalis mit Ankern befestigtes Seil nach "vorne" zu dem Operierenden herausgeleitet werden, das dann von dem Operierenden verspannt und verbunden werden kann. Es ist ebenfalls möglich die Bruchstelle so mit zwei Seilen und vier Ankerstücken, die sich nur leicht überkreuzen zu befestigen. Es ist möglich dass das Formstück zwei Perforationen im mittleren Bereich aufweist, durch die das laterale bzw. mediale Ende des Seils bzw. Drahts die bereits in der lateralen bzw. medialen Kortikalis mit Ankern befestigt sind, nach vorn ausgeleitet, verspannt und verbunden werden können.

In einer weiteren beispielhaften Ausführungsform des Implantats weist das Formstück eine Netzoberfläche auf. Das Formstück kann gegebenenfalls an der Oberfläche mit einem Netz aus resorbierbarem Material versehen sein, um die Bruchfläche besser stabilisieren zu können.

In einer anderen beispielhaften Ausführungsform des Implantats ist ein Ende des Formstücks verdickt ausgebildet. Diese Ausführungsform bildet einen resorbierbaren Marknagel der mit einer Durchgangsbohrung in Längsrichtung versehen ist. Das Formstück kann an einer Seite an dem es verdickt ist aus dem Knochen wie ein Marknagel herausstehen. Durch die Durchgangsbohrung kann ein Seil mit zwei Ankern bzw. Ankerstücken gezogen werden. Ein Anker kann das Seil direkt an bzw. in dem verdickten Formstück verankern. Der verdickte Teil kann dabei wie ein Nagelkopf wirken, der verhindert, dass das Formstück in den Knochen gezogen werden kann. In dieser Ausführungsform weist das wie ein Marknagel geformte Formstück ein Durchgangsloch in Längsrichtung auf, durch das ein Seil gezogen werden kann.

Gemäß einer anderen Ausführungsform der vorliegenden Erfindung wird ein resorbierbarer Marknagel zum Verbinden zweier Teile eines gebrochenen Röhrenknochens bereitgestellt, wobei der Marknagel an einem Ende einen verdickten Bereich und am anderen Ende eine Befestigungseinrichtung für ein Seil aufweist. Der verdickte Bereich dient dabei als Nagelkopf der verhindert, dass der Nagel weiter in den Markraum rutschen kann. Der verdickte Bereich kann als Flachkopf Rundkopf Linsenkopf oder Senkkopf ausgeführt sein. Die Befestigungseinrichtung für ein Seil kann als Durchgangsbohrung in Längs- bzw. Querrichtung, als Klemmbacken, ein Gewinde, eine Öse oder dergleichen ausgeführt sein. Durch eine Querbohrung oder eine Öse kann eine Seilschlaufe durchgezogen werden. Die Klemmbacken können sebstklemmend ausgeführt sein. Das Gewinde kann dazu dienen, ein in einem Gewindestück auslaufendes Seil an dem Marknagel zu befestigen.

Der resorbierbare Marknagel kann zudem am oberen Schaftbereich mit einem Gewinde versehen sein, um den Marknagel besser verankern zu können. Der Marknagel kann an dem Seil in die Markhöhle eines Knochens gezogen werden, sodass die Knochenstücke an den Bruchstellen aufeinander gepresst werden. Dabei verankern der "Nagelkopf" und ein Ankerstück das Implantat an den jeweiligen Knochenenden.

Gegebenenfalls weist das Formstück auch eine nagelartige Form mit einer Befestigungseinrichtung für ein Seil an der Nagelspitze auf. Diese Befestigungseinrichtung kann als Öse, Klemmbacken oder Schraubbefestigung ausgeführt sein. Das Formstück kann mit oder ohne Längshohlraum ausgeführt sein.

Nachdem der Formstücknagel in den Markraum des Knochens eingebracht wurde kann das Seil befestigt werden. Das Seil kann in beschriebener Weise gespannt und mit einem Anker an der Gegenseite befestigt werden.

In einer beispielhaften Ausführungsform ist der resorbierbare Marknagel an der Befestigungseinrichtung für ein Seil mit einem Seil versehen. Das Seil kann beispielsweise in das Material des Marknagels eingegossen sein. Dadurch kann ein zusätzlicher Schritt bei der Implantierung des Marknagels entfallen.

Der Marknagel oder das Formteil mit einem integrierten Anker ist so ausgeführt, sodass das Seil nur an einem Ende des Formstücks an- bzw. eingehängt und außerhalb der Kortikalis mittels eines zweiten Ankers verspannt und "verplombt" werden kann.

Gemäß einer Anwendung der vorliegenden Erfindung wird ein Verfahren zum Implantieren eines Implantats zum Verbinden der Teile eines gebrochenen Röhrenknochens bereitgestellt. Das Implantat umfasst dabei zwei Ankerteile, ein Seil und ein resorbierbares Formstück. Die Ankerteile können an mindestens einem Seil befestigt werden. Das Formstück weist mindestens eine Durchgangsöffnung auf, durch die das Seil geführt werden kann, sodass sich das Formstück zwischen zwei Ankerteilen um das Seil erstreckt.

Das Verfahren umfasst das Einbringen bzw. Bohren einer ersten Öffnung in den ersten Teil des gebrochenen Röhrenknochens, die von einem ersten Endbereich des Röhrenknochens durch die Wand des Röhrenknochens zu dem Bruchende innerhalb des Röhrenknochens verläuft. Weiterhin wird eine zweite Öffnung in einen zweiten Teils des gebrochenen Röhrenknochens von einem zweiten Endbereich des Röhrenknochens durch die Wand des Röhrenknochens zu dem Bruchende innerhalb des Röhrenknochens eingebracht.

Das Verfahren umfasst darüber hinaus weiter das Fädeln des Seils und des Formstücks durch die erste Öffnung, wobei das Seil durch das Formstück verläuft.

Das Verfahren umfasst weiterhin das Durchfädeln des Seils durch die zweite Öffnung, sodass das Formstück zwischen den Bruchflächen des Röhrenknochens auf das Seil aufgefädelt ist. Das Durchfädeln wird ausgeführt, sodass das Formstück zwischen den Bruchflächen des Röhrenknochens auf das Seil aufgefädelt ist. Es ist dabei insbesondere Möglich (wenn die erste Öffnung groß genug ist) das Formstück durch die erste Öffnung von dem ersten Ende des Knochens in den Markraum in Richtung der Bruchflächen einzuziehen.

Das Formteil kann in dem Verfahren auch durch die zweite Öffnung oder durch die Bruchflächen in den Markraum des Röhrenknochens eingebracht werden.

In dem Verfahren wird das Seil gestrafft und mit Ankerteilen am Knochen befestigt.

In dem Verfahren wird das Seil am ersten Endbereich des Röhrenknochens mithilfe eines ersten Ankerteils befestigt.

Schließlich wird das Seil gestrafft und am zweiten Endbereich des Röhrenknochens mithilfe eines zweiten Ankerteils befestigt.

Es ist zu Bemerken das die Ankerteile eine unterschiedliche Größe aufweisen können, falls beispielsweise die erste Öffnung größer als die zweite Öffnung ist.

Es ist anzumerken, dass das vorstehende Verfahren leicht durch das Verändern der Reihenfolge abgewandelt werden kann.

Es ist aber auch denkbar, die gesamte Operation bei geschlossener Frakturreposition über nur einen kleinen OP-Zugang am Endbereich des ersten oder zweiten Teils des Röhrenknochens zu tätigen.

Es ist damit klar, dass letztendlich nur die Reihenfolge der meisten Verfahrensschritte vertauscht werden kann.

Die eingebrachte Öffnung kann je nach Knochenzustand von der Bruchstelle aus durch den Markraum zu einem (nicht gebrochenen) Endbereich des Röhrenknochens und dort durch die Wand des Röhrenknochens gestochen gebohrt oder gefräst werden. Es ist auch möglich, die Bohrung in entgegengesetzter Richtung auszuführen. Die Öffnung kann gebohrt, gestochen gefräst oder auf andere Weise erzeugt werden. Die Öffnung kann gerade verlaufen oder zumindest teilweise gekrümmt vorgesehen sein.

Es kann notwendig sein, den Durchbruch durch die Knochenwand vor dem Endbereich des Knochens auszuführen. Der Endbereich des Knochens sei daher explizit als ein Bereich definiert, der nicht direkt an der Bruchstelle liegt, und ausreichende Befestigungsmöglichkeiten für das Ankerstück bietet.

Das Seil ist dazu bestimmt, im Inneren des Markraums zu verlaufen. Um es später zu ermöglichen, das Seil wieder zu entfernen, ist es vorgesehen, zumindest ein Ankerstück so an dem Seil zu befestigen, dass es leicht von dem Seil getrennt werden kann.

In diesem Verfahren kann auf eine Öffnung der Haut im Bruchbereich und eine Freilegung der Bruchflächen verzichtet werden. Dieses Verfahren kann insbesondere angewendet werden wenn eine geschlossene Einrichtung (reposition) des Knochenbruchs gelingt. Das Implantat kann nach einer Hautöffnung und einer Bohrung über den medialen oder lateralen Knochenpol eingebracht werden. Das eingebrachte Implantat wird dann verspannt und die Seilenden verankert und die Haut über den Einschnitten verschlossen.

Da sich die Schlüsselbeinenden trompetenförmig aufweiten können, ist es bei einer Klavikulafraktur auch möglich, vom Bruchende aus einen sich selbst verklemmenden intramedullären Anker mit anhängendem Seil zur Verspannung des Formstücks nach medial und/oder lateral (also zum Knochenende) vorzuschieben, zu platzieren den Selbstverklemmmechanismus des Ankers auszulösen und das Seil zur weiteren Operation zu straffen. Eine Eröffnung der Wand des Knochenendes wäre somit nicht notwendig. Dadurch vereinfacht sich die Operation und verkürzt sich die Operationszeit. Dieses intramedulläre Ankerprinzip kann mindestens an einem der beiden Knocheneden angewendet werden.

Gemäß einer anderen beispielhaften Anwendung der Erfindung wird ein Verfahren zum Implantieren eines Implantats zum Verbinden der Teile eines gebrochenen Röhrenknochens bereitgestellt.

Das Implantat umfasst dabei zwei Ankerteile, ein Seil und ein Formstück. Die Ankerteile können an mindestens einem Seil befestigt werden. Das Formstück weist mindestens eine Durchgangsöffnung auf, durch die das Seil geführt werden kann, sodass sich das Formstück zwischen zwei Ankerteilen um das Seil erstreckt.

Das Verfahren kann dabei das Freilegen des ersten Bruchendes eines ersten Teils eines im Wesentlichen glatt gebrochenen Röhrenknochens und das Freilegen des zweiten Bruchendes eines zweiten Teils des im Wesentlichen glatt gebrochenen Röhrenknochens umfassen. Das Verfahren kann weiter das Einbringen bzw. Bohren einer ersten Öffnung in den ersten Teil, die von dem Bruchende innerhalb des Röhrenknochens zu einem ersten Endbereich des Röhrenknochens und dort durch die Wand des Röhrenknochens verläuft, und das Einbringen einer zweiten Öffnung eines zweiten Teil die von dem Bruchende innerhalb des Röhrenknochens zu einem zweiten Endbereich des Röhrenknochens und dort durch die Wand des Röhrenknochens verläuft, umfassen.

Das Verfahren umfasst darüber hinaus weiter das Durchfädeln des Seils durch die erste Öffnung, das Durchfädeln des Seils durch das Formstück, und das Durchfädeln des Seils durch die zweite Öffnung. Das Durchfädeln wird ausgeführt, sodass das Formstück zwischen den Bruchflächen des Röhrenknochens auf das Seil aufgefädelt ist.

Das Formteil wird in dieser Ausführungsform des Verfahrens durch die Bruchflächen in den Markraum des Röhrenknochens eingebracht.

In dem Verfahren wird das Seil am ersten Endbereich des Röhrenknochens mithilfe eines ersten Ankerteils befestigt.

Schließlich wird das Seil gestrafft und am zweiten Endbereich des Röhrenknochens mithilfe eines zweiten Ankerteils befestigt.

Es ist anzumerken, dass das vorstehende Verfahren leicht durch das Verändern der Reihenfolge abgewandelt werden kann.

So kann beispielsweise erst das erste Bruchende freigelegt werden, und dann die erste Öffnung in den ersten Teil des Röhrenknochens und durch die Wand des Röhrenknochens gebohrt werden. Danach kann das Seil durch die erste Öffnung und dem Formstück gefädelt werden. Dann wird das Formteil durch die Bruchflächen in den Markraum des ersten Teils des Röhrenknochens eingebracht, oder es kann zuerst das Seil am ersten Endbereich des Röhrenknochens mithilfe eines ersten Ankerteils befestigt werden.

Es ist aber auch denkbar, die gesamte Operation bei geschlossener Frakturreposition über nur einen kleinen OP-Zugang am Endbereich des ersten oder zweiten Teils des Röhrenknochens zu tätigen.

Es ist damit klar, dass letztendlich nur die Reihenfolge der meisten Verfahrensschritte vertauscht werden kann.

Es ist ebenfalls zu bemerken, dass sich die genannten Verfahren auch für nicht medizinische Zwecke und nicht veterinäre Zwecke, wie beispielsweise für die erweiterte Leichenpräparation bei verunfallten Verstorbenen oder der Präparation allgemein eignen können. Diese spezielle Anwendung des vorliegenden Verfahrens kann in diesem speziellen Anwendungsfall nicht als eine Behandlung des Körpers gewertet werden, da bei einem bereits verstorbenen Körper keine therapeutische Behandlung im Sinne des Gesetzes möglich ist, da dieser spezielle "post mortale" Anwendungsfall nicht als Behandlung aufgefasst werden kann, die dazu dient, die Symptome einer Funktionsstörung oder Funktionsschwäche des menschlichen oder tierischen Körpers zu heilen, zu lindern oder zu beseitigen, oder abzuschwächen, oder die dazu geeignet ist, dem Risiko ihres Erwerbs vorzubeugen oder dieses zu verringern. Es ist ebenfalls möglich das Verfahren und das Implantat zur Ausbildung von Medizinern an Präparaten oder Simulatoren oder Tierknochen zu verwenden.

Die eingebrachte Öffnung kann je nach Knochenzustand von der Bruchstelle aus durch den Markraum zu einem (nicht gebrochenen) Endbereich des Röhrenknochens und dort durch die Wand des Röhrenknochens gestochen gebohrt oder gefräst werden. Es ist auch möglich, die Bohrung in entgegengesetzter Richtung auszuführen. Die Öffnung kann gebohrt, gestochen gefräst oder auf andere Weise erzeugt werden. Die Öffnung kann gerade verlaufen oder zumindest teilweise gekrümmt vorgesehen sein.

Es kann notwendig sein, den Durchbruch durch die Knochenwand vor dem Endbereich des Knochens auszuführen. Der Endbereich des Knochens sei daher explizit als ein Bereich definiert, der nicht direkt an der Bruchstelle liegt, und ausreichende Befestigungsmöglichkeiten für das Ankerstück bietet.

Das Seil ist dazu bestimmt, im Inneren des Markraums zu verlaufen. Um es später zu ermöglichen das Seil wieder zu entfernen, ist es vorgesehen, zumindest ein Ankerstück so an dem Seil zu befestigen, dass es leicht von dem Seil getrennt werden kann.

Gemäß der vorliegenden Erfindung ist das Formstück aus einem resorbierbaren Material gefertigt.

In einer weiteren beispielhaften Ausführungsform umfasst das Verfahren weiterhin das Ausrichten des Formstücks beim Einbringen des Formteils durch die Bruchflächen in den Markraum des Röhrenknochens. Damit kann beispielsweise ein Formstück mit angepasstem Querschnitt genau ausgerichtet werden, bevor die Knochenenden zusammengefügt werden. Bei der Verwendung eines Formstücks mit angepasstem Querschnitt kann sowohl eine Ausrichtung in Längsrichtung, als auch einem Winkel in axialer Richtung erreicht werden.

Das Formteil wird in dem Verfahren durch die Bruchflächen in den Markraum des Röhrenknochens eingebracht, wobei sich eine Hälfte des Formteils in den ersten Teil und die andere Hälfte des Formteils in den zweiten Teil des Röhrenknochens erstrecken soll.

Das kann in einer weiteren beispielhaften Ausführungsform das Anbringen von Schnitten zum Freilegen der Bruchstellen und der Öffnungen an der Stelle der Endbereiche umfassen, an denen die Öffnung durch die Wand des Röhrenknochens verläuft. Damit können bei Brüchen, die nicht offen sind, die jeweiligen Arbeitsbereiche freigelegt werden. Bei der vorgeschlagenen Führung der Bohrung von der Bruchstelle aus kann die Haut an der Austrittsstelle bereits durch das Bohrwerkzeug penetriert werden, eine Freilegung der Austrittsstelle ist dann ebenfalls nützlich.

Wenn die Bohrung von dem Endbereich zur Bruchstelle geführt wird, kann die Bohrstelle vorher freigelegt werden, um Komplikationen und die Zerstörung von Blutgefäßen und Nervenbahnen durch den Bohrer zu vermeiden.

Es ist ebenfalls in einer beispielhaften Form des Verfahrens vorgesehen, die Schnitte nach dem Straffen und dem Befestigen des Seils zu verschließen.

Gemäß einer weiteren Anwendung der vorliegenden Erfindung wird ein Verfahren zum Entfernen eines in der vorstehenden Beschreibung beschriebenen Implantats bereitgestellt. Sofern das Seil auch aus resorbierbarem Material besteht und somit die Materialentfernung nur die Ankerstücke betrifft. Falls das Seil und die Ankerstücke aus einem resorbierbaren Material bestehen kann eine Materialentfernung gänzlich entfallen. Das Verfahren umfasst im Einzelnen: Freilegen des ersten Ankerteils und Freilegen des zweiten Ankerteils. Das Verfahren umfasst weiterhin das Lösen des ersten oder des zweiten Ankerteils von dem Seil. Das Verfahren umfasst weiterhin das Entfernen des Seils durch Durchziehen des gelösten Endes des Seils durch die Wand des Röhrenknochens, durch den Markraum und durch die Durchgangsöffnung des Formteils und durch die Wand des Röhrenknochens. Der Formkörper verbleibt gegebenenfalls dabei im Markraum des Röhrenknochens (sofern er nicht bereits vollständig resorbiert wurde).

Das erste oder das zweite Ankerteil kann von dem Seil durch Lösen einer Schraube oder einfach mittels Durchtrennen des Seils zwischen Ankerstück und Knochen erfolgen.

Das Lösen des ersten oder des zweiten Ankerteils kann das Entfernen des Ankerteils vom Knochen selbst umfassen.

Das Durchziehen des gelösten Endes des Seils durch die Wand des Röhrenknochens kann durch Erfassen und Anziehen an dem anderen nicht gelösten Ankerteil erfolgen.

Der Formkörper kann bei dem Entfernen des Seils bereits ganz oder teilweise resorbiert sein, und verbleibt dabei im Markraum des Röhrenknochens, an welcher Stelle er dann (weiter) resorbiert wird.

In einem beispielhaften Verfahren umfasst das Freilegen der Ankerteile das Anbringen von Schnitten (durch die Haut, die über den Ankerteilen liegt), und weiter das Schließen der Schnitte nach dem Entfernen des Seils (und dem entfernen der Ankerstücke).

Der Formkörper gestattet es insbesondere, das Seil oder den Draht ohne weitere Komplikationen und ohne zusätzliche Verletzungen des Marks aus dem Knochen zu ziehen.

Nach etwa 9 bis 18 Monaten nach dem Einsetzen des Implantats können das Seil und die Ankerstücke entfernt werden. Dazu werden an beiden Enden des Seils die Ankerstücke freigelegt. Das Freilegen kann durch Einschnitte in die Haut von jeweils ca. 10 mm durchgeführt werden. Das Kabelschloss oder das Ankerstück kann an einem Ende des Seils abgetrennt (abgezwickt) werden. Das Seil kann dann an dem gegenüber liegenden Ankerstück herausgezogen werden. Danach können die Schnitte mit einer Hautnaht oder dergleichen verschlossen werden.

Gemäß einer anderen Anwendung der vorliegenden Erfindung wird ein Verfahren zum Implantieren eines Implantats zum Verbinden der Teile eines gebrochenen Röhrenknochens, bereitgestellt. Das Implantat umfasst dabei einen Marknagel mit einem verdickten Bereich an einem Ende und einer Befestigungseinrichtung für ein Seil am anderen Ende, einem Seil und einem Ankerteil.

Das Ankerteil kann an dem mindestens einem Seil befestigt werden. Das Seil kann an der Befestigungseinrichtung des Marknagels befestigt werden. Dadurch kann sich das Seil zwischen einem Ankerteil und dem Marknagel erstrecken.

Das Verfahren umfasst dabei das Zugänglichmachen des ersten Knochenendes eines ersten Teils eines im Wesentlichen glatt gebrochenen Röhrenknochens und weiter das Zugänglichmachen des zweiten Knochenendes eines zweiten Teils des im Wesentlichen glatt gebrochenen Röhrenknochens. Eine erste Öffnung wird in das erste Knochenende des ersten Teils, die von einem ersten Endbereich des Röhrenknochens und dort durch die Wand des Röhrenknochens in Richtung des Bruchendes innerhalb des Röhrenknochens verläuft eingebracht. Diese Öffnung kann sich auch über das Bruchende hinaus zum teil in den zweiten teil des Knochens erstrecken. Es ist ebenfalls möglich diese Öffnung von (einem Zugänglich gemachten) Bruchende aus zu durch den Knochen zu eingebracht werden.

Eine zweite Öffnung wird in einen zweiten Teils des Röhrenknochens von einem zweiten Endbereich des Röhrenknochens, durch die Wand des Röhrenknochens in Richtung des, ersten Endbereichs des Röhrenknochens verläuft eingebracht. Es sollte explizit darauf hingewiesen werden, dass es nicht auf die Reihenfolge ankommt, in der die erste und die zweite Öffnung jeweils an dem Knochen angebracht werden angebracht werden. Prinzipiell könnten beide Öffnungen auch gleichzeitig angebracht werden, oder die zweite Öffnung vor der ersten Öffnung. Die Begriffe erste Öffnung und zweit Öffnung betreffen im Falle des Nagels (mit Nagelkopf) hier jeweils nur die Abmessungen, der Öffnungen, wobei die erste Öffnung den Marknagel aufnehmen kann und die zweite Öffnung kleiner sein kann, um nur das Seil aufzunehmen.

Der Marknagel wird durch die erste Öffnung gefädelt bzw. in die erste Öffnung eingesteckt. Möglicherweise nach dem ein, an dem Marknagel befestigtes Seil durch die erste Öffnung gefädelt wurde. Es wird jedoch auch erwogen, den Marknagel in dem Bereich einer (möglicherweise eröffneten) Bruchstelle an dem Seil zu befestigen und so das Seil nicht durch die erste Öffnung zu fädeln. Es wird dabei ebenfalls erwogen, die eine Öffnung vom Knochenende und eine Öffnung von der Bruchstelle aus anzubringen.

Das Seil wird durch die zweite Öffnung gefädelt. Das Seil kann zuerst auch durch beide Öffnungen gefädelt werden und an dem Marknagel befestigt werden, wodurch der Marknagel an dem Seil durch den Knochen gezogen werden kann.

Der Marknagel wird weiter durch die erste Öffnung und durch die Bruchflächen in Richtung des zweiten Endbereichs des Röhrenknochens in den Knochen eingebracht bzw. eingeschoben.

Der Marknagel wird mit dem verdickten Bereich am ersten Endbereich des Röhrenknochens befestigt. Dieses Befestigen kann beispielsweise durch Festziehen des verdickten Endes an dem Knochen durchgeführt werden.

Das Seil gespannt bzw. gestrafft und am zweiten Endbereich des Röhrenknochens mithilfe eines zweiten Ankerteils befestigt. Dieses Verfahren kommt ohne eine Öffnung der Knochenbruchstelle aus.

Gemäß einer weiteren Anwendung der vorliegenden Erfindung wird ein Verfahren zum Implantieren eines Implantats zum Verbinden der Teile eines gebrochenen Röhrenknochens, bereitgestellt. Das Implantat umfasst dabei einen Marknagel mit einem verdickten Bereich an einem Ende und einer Befestigungseinrichtung für ein Seil am anderen Ende, einem Seil und einem Ankerteil.

Das Ankerteil kann an dem mindestens einem Seil befestigt werden. Das Seil kann an der Befestigungseinrichtung des Marknagels befestigt werden. Dadurch kann sich das Seil zwischen einem Ankerteil und dem Marknagel erstrecken.

Das Verfahren umfasst dabei das Freilegen des ersten Bruchendes eines ersten Teils eines im Wesentlichen glatt gebrochenen Röhrenknochens, das Einbringen einer ersten Öffnung in den ersten Teil, die von dem Bruchende innerhalb des Röhrenknochens zu einem ersten Endbereich des Röhrenknochens und dort durch die Wand des Röhrenknochens verläuft, das Freilegen des zweiten Bruchendes eines zweiten Teils des im Wesentlichen glatt gebrochenen Röhrenknochens, das Einbringen einer zweiten Öffnung eines zweiten Teils die von dem Bruchende innerhalb des Röhrenknochens zu einem zweiten Endbereich des Röhrenknochens und dort durch die Wand des Röhrenknochens verläuft, das Durchfädeln des Marknagels durch die erste Öffnung, das Durchfädeln des Seils durch die zweite Öffnung, das Einbringen des Marknagels durch die Bruchflächen in Richtung des zweiten Endbereichs des Röhrenknochens, das Befestigen des Marknagels mit dem verdickten Bereich am ersten Endbereich des Röhrenknochens, das Straffen und Befestigen des Seils, und das Befestigen des Seils am zweiten Endbereich des Röhrenknochens mithilfe eines zweiten Ankerteils.

In einer beispielhaften Form umfasst das Verfahren weiter das Befestigen des Seils an der Befestigungseinrichtung des Marknagels. Dieser Schritt wird nur notwendig, wenn nicht bereits ein Marknagel mit integriertem Seil verwendet wird. Im Prinzip werden die beiden Knochenteile auf den Marknagel gefädelt und an der anderen Seite durch das Seil und das Ankerstück fixiert. Durch das Seil und das Ankerstück kann die Bruchstelle zusammengepresst werden, was den Heilungsprozess beschleunigen kann.

Die Erfindung wird im Folgenden anhand von Ausführungsbeispielen eines teilresorbierbaren Implantats zur Osteosynthese (d. h. Heilung von Knochenbrüchen) für eine Klavikulafraktur (d.h. Schlüsselbeinbruch) mittels der Figuren. 1 bis 11 näher erläutert.
Figur 1 stellt einen Formkörper gemäß einer Ausführungsform der vorliegenden Erfindung dar.
Figur 2 ist eine Darstellung eines Seils mit einem einseitig angebrachten Ankerstück.
Figuren 3A und 3B zeigen schematisch und an einem anatomischen Präparat einen Einschnitt über einem gebrochenen Schlüsselbein.
Figuren 4A und 4B zeigen schematisch und an einem anatomischen Präparat das Einbringen der ersten Öffnung in den ersten Teil eines gebrochenen Schlüsselbeinknochens.
Figuren 5A und 5B zeigen schematisch und an einem anatomischen Präparat das Durchfädeln eines Seils mit angebrachtem Ankerstück durch die in Figuren 4A und 4B eingebrachte Bohrung.
Figuren 6A und 6B zeigen schematisch und an einem anatomischen Präparat das Durchfädeln eines Seils durch das Formstück.
Figuren 7A und 7B zeigen schematisch und an einem anatomischen Präparat das Einbringen der zweiten Öffnung in den zweiten Teil eines gebrochenen Schlüsselbeinknochens.
Figuren 8A und 8B zeigen schematisch und an einem anatomischen Präparat das Durchfädeln eines Seils durch die in Figuren 7A und 7B eingebrachte Bohrung was einem Umsetzen des Hohlbohrers entsprechen kann.
Figuren 9A und 9B zeigen schematisch und an einem anatomischen Präparat das durchgefädelte Seil und das in den Markraum eingeführte Formstück.
Figuren 10A und 10B zeigen schematisch und an einem anatomischen Präparat das Straffen des Seils und das Anbringen des zweiten Ankerstücks an dem Seil.
Figuren 11A und 11B zeigen in einer Röntgenaufnahme und an einem freigelegten anatomischen Präparat das implantierte Implantat.
Figuren 12A und 12B zeigen zwei schematische Ansichten eines Formstücks mit im Wesentlichen sternförmigem Querschnitt.
Figuren 13 bis 15 zeigen schematische Ansichten unterschiedlicher erfindungsgemäßer Ausführungsformen von Formstücken und Marknägeln.

Zur Verdeutlichung der vorliegenden Erfindung und zur Verdeutlichung des Einsatzes der Erfindung wird hier beispielhaft die Erfindung an einem Schlüsselbeinbruch verwendet. Es ist jedoch klar, dass die Erfindung prinzipiell bei jeder Art von im Wesentlichen glatten Brüchen an Röhrenknochen eingesetzt werden kann.

Üblicherweise werden Schlüsselbeinbrüche konservativ durch ca. 6-wöchige Ruhigstellung mittels Rucksackverband behandelt. Neben den zum Teil erheblich störenden Nebenwirkungen des Rucksackverbandes kann es zum Zusammenwachsen in Fehlstellung kommen.

Schlüsselbeinbrüche mit anfänglich stark ausgeprägter Fehlstellung werden daher zunehmend operativ stabilisiert. Dabei werden konventionell die gebrochenen Enden durch eine verschraubte Platte d.h. mit einer "Plattenosteosynthese" verbunden. Alternativ können Schlüsselbeinbrüche auch durch einen Marknagel verbunden werden.

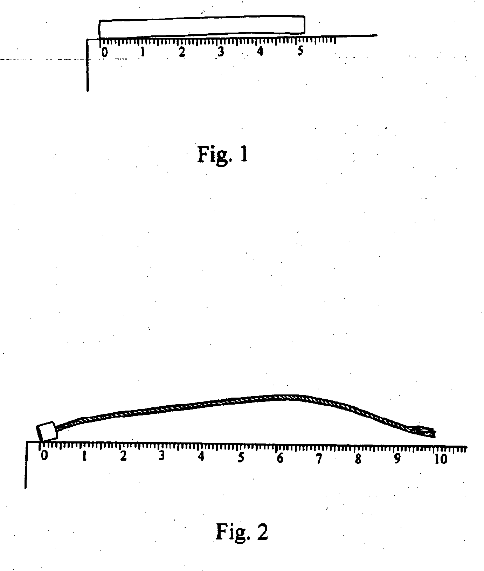

Figur 1 stellt einen Formkörper gemäß einer Ausführungsform der vorliegenden Erfindung dar.

Das Implantat besteht im Wesentlichen aus zwei Teilen und ist daher sehr einfach in der operativen Handhabung, wobei Modifikationen möglich sind.

Der erste Teil besteht aus einem resorbierbaren Formstück, das in der Figur 1 als Schlauch ausgeführt ist. Der Schlauch ist resorbierbar. Der dargestellte Schlauch weist eine (End-) Länge von ca. 80 mm und einen Innendurchmesser von ca. 2 mm auf. Der Außendurchmesser beträgt ca. 4,5 mm. Der in Figur 1 dargestellte resorbierbare Schlauch ist für Brüche des Schlüsselbeins ausgelegt. Durch den Schlauch kann ein Draht, ein resorbierbares Seil oder ein Drahtseil gefädelt werden. Die Reiß- und Biegebelastbarkeit entspricht in etwa einem herkömmlichen Antennenkabel. Die Außenoberfläche des resorbierbaren Schlauchs kann profiliert sein. Die Abmessungen und das Material können so gewählt werden, dass sich eine Resorptionszeit von ca. 12 Wochen oder länger ergibt.

Figur 2 ist eine Darstellung, die ein Seil mit einem einseitig angebrachten Ankerstück darstellt. Das Drahtseil kann ca. 20 cm lang sein und eine Stärke bzw. einen Durchmesser von ca. 1 bis ca. 2 mm aufweisen. Der Durchmesser kann je nach Anwendungsfall, d.h. den Abmessungen des jeweiligen Knochens, variiert werden. Das Seil ist an einem Draht- bzw. Seilende mit einem Drahtschloss als Ankerstück versehen. Das andere Seilende kann, nach dem Durchziehen durch den Knochen, mit einem weiteren Drahtschloss als Ankerstück an dem Knochen befestigt werden. Dabei kann ein Drahtschloss bereits am Kabelende fixiert sein. Das zweite Schloss (d.h. Ankerstück) wird erst am Ende der Operation auf den Draht gefädelt und dann (z.B. ähnlich einer Plombe) verschlossen.

Es ist ebenfalls vorgesehen, ein geflochtenes (mehrlitziges) Seil oder ein monofiles Seil (d.h. einen Draht) zu verwenden. Es kann ebenfalls vorgesehen werden, ein im Querschnitt rundes oder flaches bzw. bandförmiges Seil zu verwenden. Das Seil und/oder die Ankerstücke können aus resorbierbarem Material gefertigt sein.

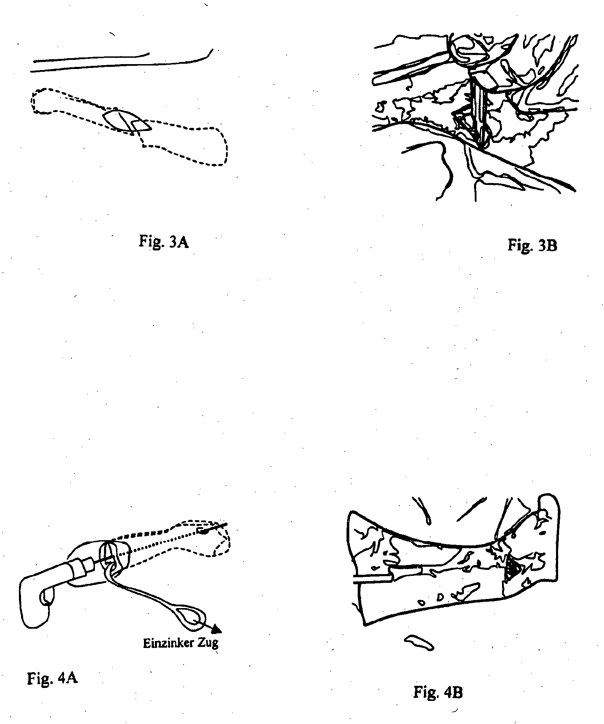

Figuren 3A und 3B zeigen schematisch und an einem anatomischen Präparat einen Einschnitt über einem gebrochenen Schlüsselbein. Dazu wird ein Hautschnitt von etwa 2 cm Länge über dem Bruch- bzw. Frakturbereich angebracht.

Figuren 4A und 4B zeigen schematisch und an einem anatomischen Präparat das Einbringen der ersten Öffnung in den ersten Teil eines gebrochenen Schlüsselbeinknochens. Dies kann durch Aufbohren des medialen Fragmentes bis ca. 4 cm mit einem Durchmesser von ca. 5 mm durchgeführt werden. Diese Bohrung kann mit einem Hohlbohrer von ca. 2 mm Durchmesser überbohrt werden. Diese Bohrung durchringt das Ende des Knochens mit einer gebohrten medio-ventralen Kortikalisperforatiön. Die Bohrung kann ebenfalls die Haut perforieren. Es ist möglich für diesen Schritt einen Stufen-Hohlbohrer zu verwenden. An der Austrittsstelle der Überbohrung aus dem Knochen wird ein ca. 5 mm langer Hautschnitt angebracht.

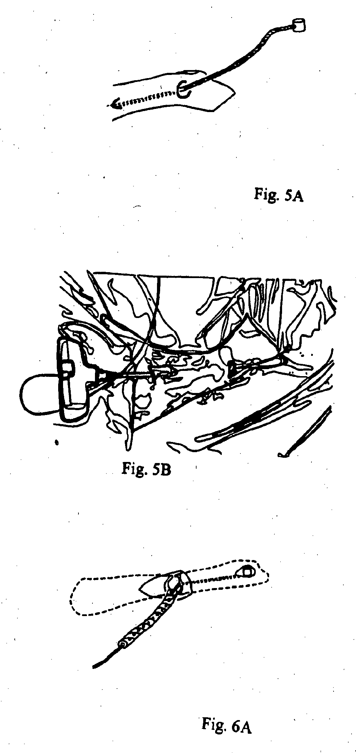

Figuren 5A und 5B zeigen schematisch und an einem anatomischen Präparat das Durchfädeln eines Seils mit angebrachtem Ankerstück durch die in Figuren 4A und 4B eingebrachte Bohrung. Das Einfädeln des Kabeldrahtes von medial kann durch bzw. über Hohlbohrer erfolgen. Der Draht wird bis zur Bruchstelle durchgezogen. Der Hohlbohrer wird entfernt.

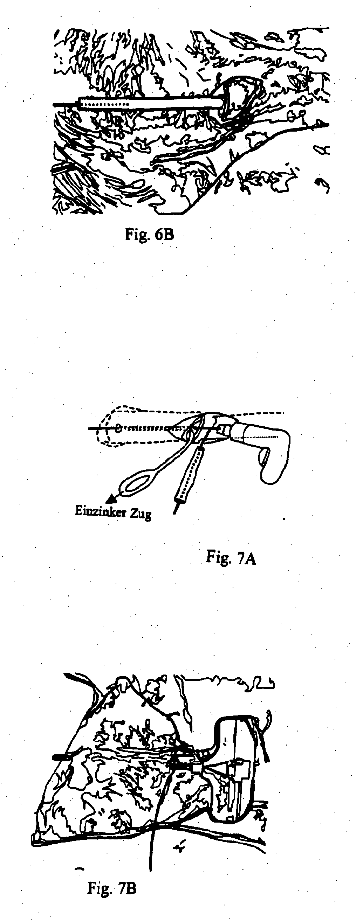

Figuren 6A und 6B zeigen schematisch und an einem anatomischen Präparat das Durchfädeln eines Seils durch das Formstück. Das resorbierbare Formstück wird an der Bruchstelle auf das Kabel bzw. das Seil aufgezogen. Das resorbierbare Formstück wird über das Seil und die Bruchstelle in den Markraum des Knochens eingeschoben. Das resorbierbare Formstück kann zumindest bis zur Tiefe der ersten Bohrung (mit dem Durchmesser 5 mm) eingeschoben werden.

Es ist ebenfalls vorgesehen einen Fräser oder Schwingfräser zu verwenden, um profilierte Bohrungen und oder im Querschnitt nicht runde Bohrungen anzubringen. Dies kann insbesondere von in Längs- und Umfangsrichtung profilierten Formteilen genutzt werden.

Figuren 7A und 7B zeigen schematisch und an einem anatomischen Präparat das Einbringen der zweiten Öffnung in den zweiten Teil eines gebrochenen Schlüsselbeinknochens. Das zweite (laterale) Fragment des Knochens kann ca. 4 cm tief mit einem Durchmesser von ca. 5 mm gebohrt werden. Diese Bohrung kann latero-dorsal durch das Überbohren mit einem Hohlbohrer (mit einem Durchmesser von ca. 2 mm) durch die Knochenwand weitergeführt werden. An der Stelle, an der der Bohrer durch die Knochenwand geführt wird, kann ein ca. 0,5 cm langer Hautschnitt angebracht werden.

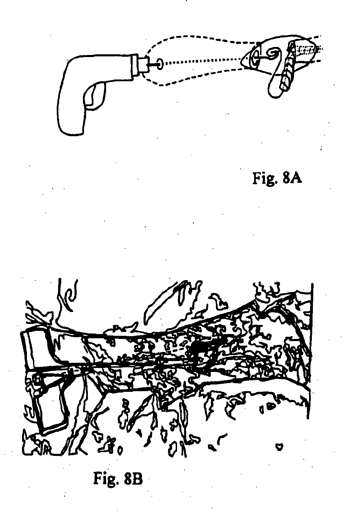

Figuren 8A und 8B zeigen schematisch und an einem anatomischen Präparat das Durchfädeln eines Seils durch die in Figuren 7A und 7B eingebrachte Bohrung. Das Seil wird von der Seite (lateral) durch die Bruchfläche über Hohlbohrer durch den Knochen gefädelt. Der Hohlbohrer wird dann entfernt.

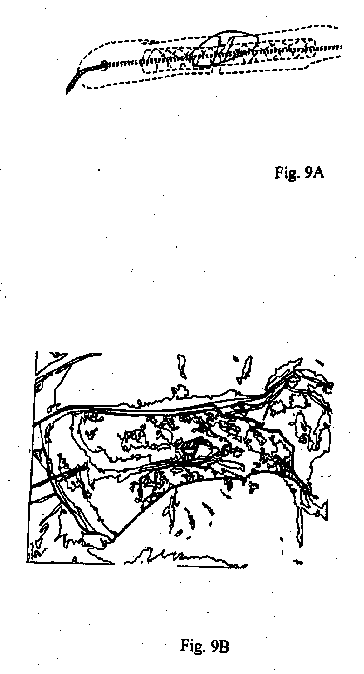

Figuren 9A und 9B zeigen schematisch und an einem anatomischen Präparat das durchgefädelte Seil und das in den Markraum eingeführte Formstück. In den Figuren wird das Formstück in dem Markraum (intermedullar) angeordnet und ausgerichtet. Dabei wird das Formstück jeweils ca. 4 cm in den Markraum der Knochenbruchstücke eingeschoben.

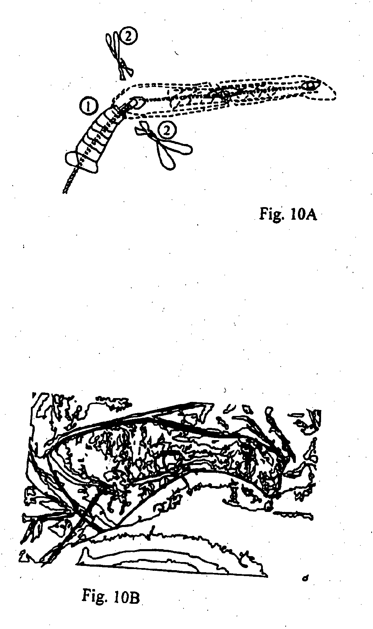

Figuren 10A und 10B zeigen schematisch und an einem anatomischen Präparat das Straffen des Seils und das Anbringen des zweiten Ankerstücks an dem Seil. Das Straffen des Seils wird durch Anspannen des Kabeldrahtes durch Zug am lateralen Ende durchgeführt, bis das erste (mediale) Kabelschloss an der anderen (medialen) Seite des Schlüsselbeins aufliegt. Dann wird das (laterale) Ankerstück in Form eines Kabelschloss auf dem Seil aufgefädelt.

Nach dem Auffädeln des lateralen Kabelschlosses wird ein Kabelspanner (bzw. ein Seilspanner) angesetzt und das Seil straff gespannt. Die genaue Spannung ergibt sich aus der jeweiligen Knochen- und Bruchsituation. Beim Anspannen des Seils werden die beiden Knochenenden unter Kontrolle der Frakturregion zusammengeführt. Bei glattem Aufliegen der Bruchstellen aneinander wird das (laterale) Kabelschloss festgeklemmt. Der Kabelspanner wird entfernt und ein überstehendes, überschüssiges Seil wird lateralseitig abgetrennt bzw. abgezwickt. Danach kann die Haut an den drei Schnitten wieder verschlossen werden.

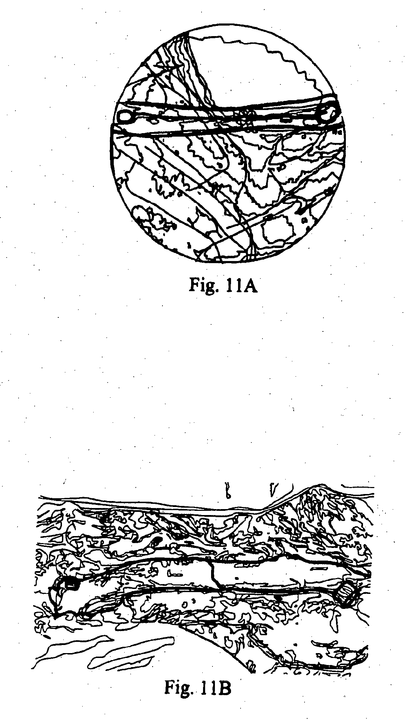

Figuren 11A und 11B zeigen in einer Röntgenaufnahme und an einem freigelegten anatomischen Präparat das eingesetzte Implantat. In der Röntgenaufnahme sind lediglich das Seil und die beiden Ankerstücke zu erkennen. Das resorbierbare Formstück ist auf der Röntgenaufnahme nicht zu erkennen. Lediglich der Verlauf des Drahtes kann anzeigen, dass das Seil nicht an der inneren Knochenwand des Markraums anliegt. An dem freigelegten Schlüsselbein des anatomischen Präparates sind lediglich die beiden Ankerstücke in Form von Kabelschlössern zu erkennen. Der Formkörper befindet sich unsichtbar im Markraum des Schlüsselbeins.

Nach Verheilen des Knochenbruchs können das Seil und die Ankerstücke entfernt werden. Dazu wird die Haut über den Ankerstücken aufgeschnitten. Die Schnitte können jeweils ca. 5 bis 10 mm lang sein. Ein Ankerstück kann durch Abtrennen bzw. Abzwicken des Seils an einem Ende entfernt werden. Das Seil kann dann an dem gegenüberliegenden Ankerstück herausgezogen werden. Die Schnitte können mit einer Hautnaht oder dergleichen verschlossen werden.

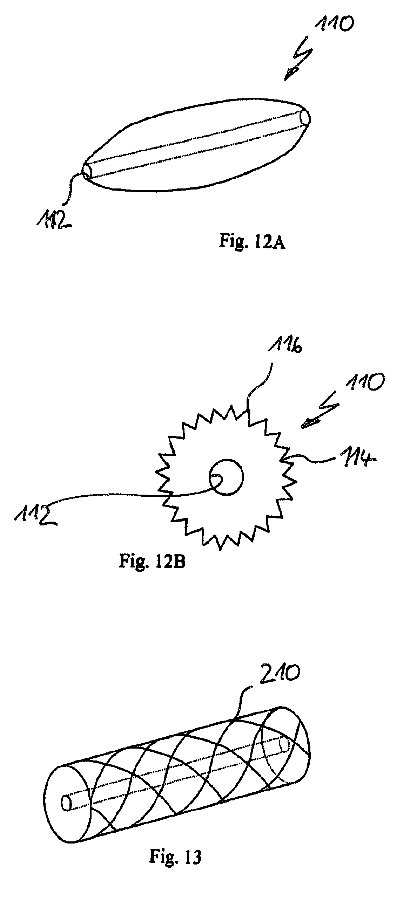

Figuren 12A und 12B zeigen eine Ausführung eines im Wesentlichem spindelförmigen resorbierbaren Formstücks in einer Seitenansicht (Fig. 12A) und einer Querschnittsansicht (Fig. 12B). Durch die Rillen bzw. die Stege oder Rippen kann sich das Formstück in dem Hohlraum des Knochens verhaken oder verkeilen sodass eine Torsionsbewegung der Bruchenden gegeneinander um die Längsachse des Formstücks nicht möglich ist. In den Figuren 12A und 12B ist das resorbierbare Formstück 110 mit einer Durchgangsbohrung bzw. Ausnehmung 112 versehen durch das dann das Seil zur Stabilisierung durchgezogen werden kann. Durch die Längsrillen 114 und die Längsstege bzw. Längsrippen 116 kann verhindert werden dass sich die beiden Knochenenden an der Bruchstelle gegeneinander verdrehen können. Bei einem elastischen Formstück kann eine im Wesentlichen Sternförmige Geometrie auch dazu dienen, das Formstück an nicht runde Knocheninnenquerschnitte anzupassen, indem sich die Stege gegeneinander legen können. Die Außenseite des Formstücks kann ebenfalls mit in radialer Richtung gebogenen oder auch spiralförmig angeordneten Lamellen versehen sein. Es ist auch möglich, dass das Formstück an der Außenseite aufgeraut ist um die Fertigung zu vereinfachen.

Figur 13 zeigt eine Ausführungsform eines Formstücks in einer schematischen Ansicht mit einer Netzoberfläche. Das Formstück kann gegebenenfalls an der Oberfläche mit einem resorbierbaren Netz 210 oder ähnlichem versehen sein, um die Bruchfläche besser stabilisieren zu können.

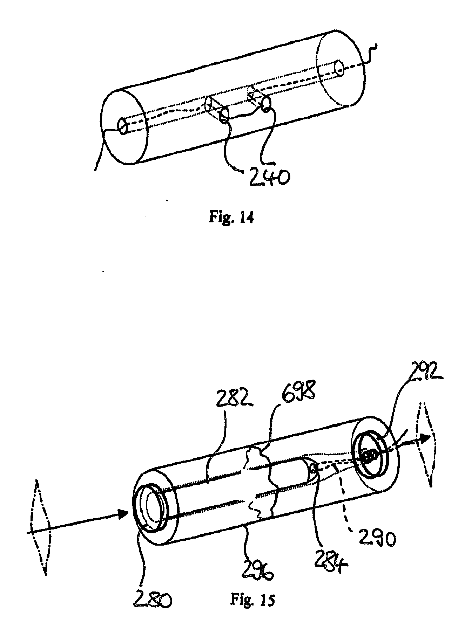

Figur 14 zeigt eine Ausführungsform eines Förmstücks in einer schematischen Ansicht mit mindestens zwei weiteren Löchern 240, die sich im Wesentlichen quer zu der Längsrichtung des Formstücks erstrecken. Dadurch kann ein in der Kortikalis mit Ankern befestigtes Seil nach "vorne" zu dem Operierenden herausgeleitet werden, das dann von dem Operierenden verspannt und verbunden werden kann. Es ist ebenfalls möglich die Bruchstelle so mit zwei Seilen und vier Ankerstücken, die sich nur leicht überkreuzen zu befestigen. Es ist möglich dass das Formstück zwei Perforationen im mittleren Bereich aufweist, durch die das laterale bzw. mediale Ende des Seils bzw. Drahts die bereits in der lateralen bzw. medialen Kortikalis mit Ankern befestigt sind, nach vorn ausgeleitet, verspannt und verbunden werden können.

Figur 15 zeigt eine schematische Ansichte einer Ausführungsform eines Marknagels. Der Marknagel umfasst dabei einen Nagelkopf 280, einen Nagelschaft 282, eine Öse 284 als Befestigungsvorrichtung für ein Seil 290. Der Marknagel wird durch das Seil und durch das Ankerstück 292, gegenüber dem Knochen verspannt wodurch der an der Bruchlinie 698 gebrochenen Knochen 296 an der Bruchlinie 698 zusammengepresst wird.

Der Marknagel kann durch einen auf der linken Seite angedeuteten Hautschnitt in Pfeilrichtung in den Knochen eingebracht werden. Das Seil kann durch einen auf der rechten Seite angedeuteten Hautschnitt in Pfeilrichtung gegenüber dem Ankerstück bzw. der Verankerung gespannt werden. Das Seil kann durch den auf der rechten Seite angedeuteten Hautschnitt an dem Ankerstück befestigt werden. Der Markraum des ersten (linken) Knochenstücks weist dabei als erste Öffnung eine gleichmäßige (Zylinder-) Bohrung auf. Der Markraum des zweiten (rechten) Knochenstücks weist dabei als zweite Öffnung eine verjüngte Bohrung auf. Die zweite Bohrung kann dabei durch die erste Bohrung in das zweite Knochenbruchstück ausgeführt werden, wodurch der Eingriff an der Seite des zweiten Knochenstücks geringer gehalten werden kann.

Damit basiert das Implantat auf einer inneren Schienung und auf einem axialen Zug, um eine sicherere Heilung des Bruchs und eine erhöhte Anfangsstabilität zu erreichen. Das Implantat dient richtig angebracht dazu, die gebrochenen Teile eines Knochens zusammenzuziehen und die Bruchflächen gegeneinander zu drücken.

Die Schienung basiert im Gegensatz zu herkömmlichen Verfahren jedoch nicht auf einem völlig starren Formteil, sondern auf einem straff gespannten Seil oder Draht, und einem den Draht umgebenden Formteil. Das Formteil kann sich dabei an dem straff gespannten Seil bzw. Draht abstützen. Dies ermöglicht eine hohe Primärstabilität und gleichzeitig ein Zusammenpressen der Bruchflächen. Durch das als Innenschiene wirkende Formteil ergibt sich eine Neutralisation von möglicherweise auftretenden Scherkräften.

Der einzigartige Spannmechanismus gestattet mit nur einer einzigen Operation eine sofortige postoperative Mobilisation des Schlüsselbeins wahrscheinlich innerhalb bestimmter Bewegungs- und Belastungsgrenzen. Durch die Verwendung des selbstauflösenden Formteils muss ein wesentlicher Teil des Implantats nicht mehr entfernt werden. Alle diese Vorteile werden dabei mit einem operativen Verfahren erreicht, dass im Verhältnis zu den konventionellen Verfahren mit nur geringem Blutverlust und kleiner Wundfläche auskommt. Dabei wird trotzdem eine stabile Bruchversorgung erreicht.

Durch die Verwendung des vorliegenden Implantats muss ein Patient nur einmal beim Einsetzten des Implantats bzw. beim Stabilisieren des Bruchs unter Vollnarkose operiert werden. Die Entfernung des Implantats nach der Heilung des Bruchs (d. h. rund 9 bis 18 Monaten nach der Implantation) kann ambulant unter Lokalanästhesie erfolgen. Damit stellt das Implantat insbesondere für den Patienten eine erhebliche Erleichterung dar.

Durch die Verwendung eines minimal invasiven Zugangs wird die Bildung von Blutgefäßen im Bereich des Bruchs erhalten, was wiederum der Heilung des Knochens zugute kommt.

Es sollte hier nochmals darauf hingewiesen werden, dass das Implantat prinzipiell bei allen orthopädischen und chirurgischen und unfallchirurgischen Behandlungen instabiler Brüche von Röhrenknochen verwendet werden kann. Es ist ebenfalls möglich dieses Verfahren und die Implantate der Erfindung in der Veterinärmedizin zu verwenden.

Der Schutzumfang dieser Erfindung wird durch die Patentansprüche und nicht lediglich durch die in der Zeichnung dargestellten Ausführungsformen bestimmt. Änderungen und Abwandlungen des Gegenstandes der Zeichnungen sollen ebenfalls in den Schutzumfang fallen, soweit sie unter den Wortlaut der angehängten Ansprüche fallen. So ist beispielsweise vorstellbar, anstelle der Kabelschlösser oder "Nippel" Ankerstücke zu verwenden, die an dem Knochen festgenagelt oder festgeschraubt werden können. Es ist ebenfalls möglich, das Seil oder Kabel mehrfach, beispielsweise in Schlingen, oder mehrere Formteile oder ein Formteil mit mehreren Durchgangsöffnungen zu verlegen. Es können auch Führungshülsen an der Stelle vorgesehen werden, an der das Seil bzw. der Draht durch die Knochenwand tritt. ,

## Patentansprüche

1. Implantat für die zugfeste Verbindung zweier Teile eines im Wesentlichen glatt gebrochenen Röhrenknochens, wobei das Implantat mindestens ein Formstück aufweist, wobei das Formstück (110) mindestens eine Durchgangsöffnung (112) aufweist und aus einem resorbierbaren Material gebildet ist, und wobei das Implantat mindestens zwei Ankerteile und mindestens ein Seil aufweist, die Ankerteile an dem mindestens einem Seil befestigt werden können und das Seil durch die mindestens eine Durchgangsöffnung geführt werden kann, sodass sich das Formstück zwischen zwei Ankerteilen um das Seil erstreckt, **dadurch gekennzeichnet, dass** das Formstück (110) dazu bestimmt ist, in Längsrichtung eines gebrochenen Röhrenknochens in den Markraum des gebrochenen Röhrenknochens eingeführt zu werden und dass entweder das Formstück (110) zylinderförmig oder im Wesentlichen zylinderförmig ausgeführt und mit abgerundeten oder konisch verlaufenden Enden versehen ist oder das Formstück (110) spindelförmig oder im Wesentlichen spindelförmig ausgeführt ist.

2. Implantat nach Anspruch 1, **dadurch gekennzeichnet, dass** das Seil ein Stahlseil ist, das aus Einzelfasern besteht.

3. Implantat nach Anspruch 1, **dadurch gekennzeichnet, dass** das Seil aus resorbierbaren Material besteht.

4. Implantat gemäß einem der Ansprüche 1 bis 3, **dadurch gekennzeichnet, dass** das Formstück (110) mit nicht kreisförmigem Querschnitt ausgeführt ist.

5. Implantat gemäß einem der Ansprüche 1 bis 4, **dadurch gekennzeichnet, dass** die Oberfläche des Formstücks (110) profiliert ist.

6. Implantat gemäß einem der Ansprüche 1 bis 5, **dadurch gekennzeichnet, dass** das Formstück (110) mit Längsrillen (114) oder mit Querrillen versehen ist.

7. Implantat gemäß einem der Ansprüche 1 bis 6, **dadurch gekennzeichnet dass** das Formstück (110) einen im Wesentlichen sternförmigen Querschnitt aufweist.

8. Implantat gemäß einem der vorstehenden Ansprüche, **dadurch gekennzeichnet, dass** das resorbierbare Material ausgewählt ist aus der Gruppe umfassend Polyglycolid (PGA), Glycolidcopolymere, Glycolide- /lactidcopolymere (PGA/PLA), Glycolid/trimethylencarbonatcopolymere (PGA/TMC), Stereoisomere und Copolymere von Polylactid, Poly-L-lactid (PLLA), Poly-D-lactid (PDLA), Poly-DL-lactid (PDLLA), L-Lactid/DL-lactidcopolymere, L-Lactid/D-lactidcopolymere, Lactid/tetramethylenglycolidcopolymere, Lactid/tri- methylencarbonatcopolymere, Lactid/δ-valerolactoncopolymere, Lactid/ε- caprolactoncopolymers, Polydepsipeptid (Glycin-DL-lactidcopolymer), Polylactid/ethylenoxidcopolymere, asymmetrisch 3,6-substituierte Poly-1,4-dioxan- 2,4-dione, Poly-β-hydroxybutyrat (PHBA), PHBA/ß-hydroxyvaleratcopolymere (PHBA/PHVA), Poly-β-hydoxypropionat (PHPA), Poly-ß-dioxanon (PDS), Poly-Δ- valerolacton, Poly-Δ-caprolacton, Methylmethacrylat-N-vinylpyrrolidoncopolymere, Polyesteramide, Polyester von Oxalsäure, Polydihydropyrane, Polyalkyl-2- cyanoacrylate, Polyurethane (PU), Polyvinylalkohol (PVA), Polypeptide aus α- Aminosäuren, Poly-ß-maleinsäure (PMLA), Poly-β-alkansäuren, Polyethylenoxid (PEO), Chitinpolymere, Calciumhydroxidapatit oder Tricalciumphosphat.

9. Implantat gemäß einem der Ansprüche 1 bis 8, **dadurch gekennzeichnet, dass** das Formstück aus einzelnen schlauch- bzw. spindelförmigen Abschnitten zusammengesetzt ist, die zumindest abschnittsweise ineinander geschoben sind.

10. Implantat gemäß Anspruch 9, **dadurch gekennzeichnet, dass** die einzelnen Abschnitte ein resorbierbares Material mit unterschiedlichen Resorptionsgeschwindigkeiten aufweisen.

11. Implantat gemäß einem der Ansprüche 1 bis 10, **dadurch gekennzeichnet, dass** das Formteil mindestens zwei weitere Löcher (240) aufweist die im Wesentlichen quer zu der Längsrichtung des Formstücks angeordnet sind.

12. Implantat gemäß einem der Ansprüche 1 bis 11, **dadurch gekennzeichnet, dass** das Formstück mit einer Netzoberfläche (210) versehen ist.

13. Implantat gemäß einem der Ansprüche 1 bis 12, **dadurch gekennzeichnet, dass** das Seil in das Material des Formstücks (110) eingegossen ist.

## Claims

1. Implant for the tension-resistant connection of two parts of a long bone that has broken with a substantially smooth break, wherein the implant has at least a shaped piece, wherein the shaped piece (110) has at least one through-opening (112) and is formed from a resorbable material, und wherein the implant has at least two anchor parts and at least one cord, the anchor parts can be secured to the at least one cord and the cord can be guided through the at least one through-opening so that the shaped piece extends around the cord between two anchor parts, **characterised in that** the shaped piece (110) is adapted for insertion into the medullar cavity of a broken long bone in the longitudinal direction of the one broken long bone, and **in that** either the shaped piece (110) is cylindrical or substantially cylindrical and provided with rounded or conically extending ends or the shaped piece (110) is in the form of a spindle or substantially in the form of a spindle.

2. Implant according to claim 1, **characterised in that** the cord is a steel cord composed of individual fibres.

3. Implant according to claim 1, **characterised in that** the cord is made of resorbable material.

4. Implant according to one of the preceding claims 1 to 3, **characterised in that** the shaped piece (110) is made with non-circular cross-section.

5. Implant according to any one of the claims 1 to 4, **characterised in that** the surface of the shaped piece (110) is contoured.

6. Implant according to any one of the claims 1 to 5, **characterised in that** the shaped piece (110) is provided with longitudinal grooves (114) or with transverse grooves.

7. Implant according to any one of the claims 1 to 6, **characterised in that** the shaped piece (110) has a substantially star-shaped cross-section.

8. Implant according to any one of the preceding claims, **characterised in that** the resorbable material is selected from the group comprising polyglycolide (PGA), glycolide copolymers, glycolide/lactide copolymers (PGA/PLA), glycolide/trimethylene carbonate copolymers (PGA/TMC), stereoisomers and copolymers of polylactide, poly-L-lactide (PLLA), poly-D-lactide (PDLA), poly-DL-lactide (PDLLA), L-lactide/DL-lactide copolymers, L-lactide/D-lactide copolymers, lactide/tetramethylene glycolide copolymers, lactide/trimethylene carbonate copolymers, lactide/δ-valerolactone copolymers, lactide/ε-caprolactone copolymers, polydepsipeptide (glycine-DL-lactide copolymer), polylactide/ethylene oxide copolymers, asymmetrically 3,6-substituted poly-1,4-dioxane-2,4-diones, poly-β-hydroxybutyrate (PHBA), PHBA/β-hydroxyvalerate copolymers (PHBA/PHVA), poly-β-hydroxypropionate (PHPA), poly-β-dioxanone (PDS), poly-Δ-valerolactone, poly-Δ-caprolactone, methyl methacrylate-N-vinyl pyrrolidone copolymers, polyester amides, oxalic acid polyesters, polydihydropyrans, polyalkyl-2-cyanoacrylates, polyurethanes (PU), polyvinyl alcohol (PVA), polypeptides from α-amino acids, poly-β-maleic acid (PMLA), poly-β-alkanoic acids, polyethylene oxide (PEO), chitin polymers, calcium hydroxide apatite or tricalcium phosphate.

9. Implant according to any one of the claims 1 to 8, **characterised in that** the shaped piece is composed of individual tubular or spindle-shaped sections, which are slid into one another at least in some sections.

10. Implant according to claim 9, **characterised in that** the individual sections have a resorbable material with different resorption rates.

11. Implant according to any one of the preceding claims 1 to 10, **characterised in that** the shaped piece has at least two further holes (240), which are arranged substantially transversely to the longitudinal direction of the shaped piece.

12. Implant according to any one of the preceding claims 1 to 11, **characterised in that** the shaped piece is provided with a mesh surface (210).

13. Implant according to any one of the preceding claims 1 to 12, **characterised in that** cord is cast into the material of the shaped piece (110).

## Revendications

1. Implant destiné à assurer la liaison de deux parties d'un os tubulaire ou os long ayant subi une fracture sensiblement lisse, l'implant présentant au moins une pièce de forme, la pièce de forme (110) présentant au moins une ouverture de passage (112) et étant réalisée en un matériau résorbable, et l'implant présentant au moins deux pièces d'ancrage et au moins un câble, implant dans lequel les pièces d'ancrage peuvent être fixées au dit au moins un câble, et le câble peut être guidé et mené au travers de ladite au moins une ouverture de passage, de sorte que la pièce de forme s'étend autour du câble entre deux pièces d'ancrage, **caractérisé en ce que** la pièce (110) est destinée à être introduite, dans la direction longitudinale d'un os tubulaire fracturé, à l'intérieur du canal médullaire de l'os tubulaire fracturé, et **en ce que**, soit la pièce de forme (110) est d'une configuration de forme cylindrique ou sensiblement de forme cylindrique et est pourvue d'extrémités arrondies ou s'étendant de manière conique, soit la pièce de forme (110) est d'une configuration en forme de fuseau ou sensiblement en forme de fuseau.

2. Implant selon la revendication 1, **caractérisé en ce que** le câble est un câble en acier, qui est constitué de fibres individuelles.

3. Implant selon la revendication 1, **caractérisé en ce que** le câble est réalisé en un matériau résorbable.

4. Implant selon l'une des revendications 1 à 3, **caractérisé en ce que** la pièce de forme (110) est réalisée avec une section transversale de forme non circulaire.

5. Implant selon l'une des revendications 1 à 4, **caractérisé en ce que** la surface externe de la pièce de forme (110) est profilée.

6. Implant selon l'une des revendications 1 à 5, **caractérisé en ce que** la pièce de forme (110) est dotée de rainures longitudinales (114) ou de rainures transversales.

7. Implant selon l'une des revendications 1 à 6, **caractérisé en ce que** la pièce de forme (110) présente une section transversale sensiblement en forme d'étoile.

8. Implant selon l'une des revendications précédentes, **caractérisé en ce que** le matériau résorbable est choisi parmi un groupe comprenant : acide polyglycolique (PGA), copolymères d'acide glycolique, copolymères d'acide glycolique/lactide (PGA/PLA), copolymères d'acide glycolique/tri-méthylène-carbonate (PGA/TMC), stéréo-isomères et copolymères poly-lactide, poly-L-lactide (PLLA), poly-D-lactide (PDLA), poly-DL-lactide (PDLLA), copolymères L-lactide/DL-lactide, copolymères L-lactide/D-lactide, copolymères lactide/ tétraméthylène-acide glycolique, copolymères lactide/tri-méthylène-carbonate, copolymères lactide/δ-valérolactone, copolymères lactide/ε-caprolactone, poly-depsipeptide (copolymères glycine-DL-lactide), copolymères polylactide/oxyde d'éthylène, poly-1,4-dioxane-2,4-dione-3,6 substitué asymétriquement, poly-β-hydroxybutyrate (PHBA), copolymères PHBA/β-hydroxyvalerate (PHBA/PHVA), poly-β-hydroxyproprionate (PHPA), poly-β-dioxanone (PDS), poly-Δ-valerolactone, poly-Δ-caprolactone, copolymères méthylméthacrylate-N-polydihydropyrane, polyalkkyle-2-cyanoacrylate, polyuréthanne (PU), alcool polyvinylique (PVA), acide amino-α-polypeptide, acide poly-β-maléique (PMLA), acide poly-β-alcane, oxyde de polyéthylène (PEO), polymères de chitine, calcium-hydroxyapatite ou tri-calcium-phosphate.

9. Implant selon l'une des revendications 1 à 8, **caractérisé en ce que** la pièce de forme est composée de tronçons individuels en forme de tuyau ou de fuseau, qui sont emmanchés au moins partiellement les uns dans les autres.

10. Implant selon la revendication 9, **caractérisé en ce que** les tronçons individuels comprennent un matériau résorbable avec des vitesses de résorption différentes.

11. Implant selon l'une des revendications 1 à 10, **caractérisé en ce que** la pièce de forme présente au moins deux autres trous (240), qui sont agencés sensiblement transversalement à la direction longitudinale de la pièce de forme.

12. Implant selon l'une des revendications 1 à 11, **caractérisé en ce que** la pièce de forme est pourvue d'une surface en forme de filet (210).

13. Implant selon l'une des revendications 1 à 12, **caractérisé en ce que** le câble est noyé par moulage dans le matériau de la pièce de forme (110).
